# EUROPEAN PATENT SPECIFICATION

(11) **EP 2 042 216 B1**
(45) Date of publication and mention of the grant of the patent: **09.09.2015**
(21) Application number: 07117550.9
(22) Date of filing: 28.09.2007
(51) Int. Cl.: A61Q 5/02, A61Q 19/10, A61K 8/37, A61K 8/49, A61K 8/34

(54) **Body and hair cleansing formulations**
Körper- und Haarreinigungsformulierungen
Formulations de nettoyage du corps et des cheveux

(43) Date of publication of application: 01.04.2009
(73) Proprietor: Omega Pharma Nordic AB, 164 07 Kista (SE)
(72) Inventor: Lodén, Marie, 170 77, Solna (SE); Buraczewska, Izabela, 111 23, Stockholm (SE); Åkerström, Ulf, 117 62, Stockholm (SE); Wärnheim, Torbjörn, 163 42, Spånga (SE)
(74) Representative: Brann AB

(56) References cited:
- EP-A- 1 043 010
- DE-C1- 4 311 535
- GB-A- 2 420 076
- JP-A- 2001 261 549
- US-A1- 2003 158 065

## Description

### Field of the invention

The present invention generally relates to body and hair cleansing products, and particularly such products containing as necessary ingredients in addition to water only substances appearing on the E list, *i*.*e*. substances approved for use as foodstuff additives. The products are especially intended for infants and children.

### Background art

Formulations for personal care are conventionally known and have been used for a very long time. Products such as shampoo and liquid soap, are well-established products belonging to this category. Generally, such products are formulated so as to have a smell which is attractive or at least agreeable to the consumer.

Such products have also been formulated especially for babies, such as baby shampoos. EP-A-0 569 028 *i*.*a*. relates to baby shampoos. For a baby shampoo mildness and low irritability are important requirements. EP-A-0 569 028 is directed to such shampoos without the need of using ethylene oxide derivatives, such as polyethylene glycol 6000 distearate, and polysorbate 20, wherein certain high molecular weight polyglyceryl esters (PGEs) are used instead. As an optional ingredient a polyol may be added, such as glycerin, propylene glycol, sorbitol or other cosmetically acceptable polyols. The polyol is suitably added in an amount of 0.5-10 wt. %, e.g. 2-2.5 wt. % in a baby shampoo. Preservatives, such as dimethyl dimethylolhydantoin (DMDMH), may optionally be included.

JP 2001 261549A relates to cleansing ingredients consisting of components such as additives for food with high oral safety. The components in the cleansing ingredient are said to have a high oral safety when accidentally ingested during use by children or elderly people. The formulation specified in the Example i.a. contains 5.0 % by weight of glycerol and 0.05 % by weight paraoxy ethyl benzoate as a preservative.

The present applicant has developed a commercial baby shampoo product containing 40 % by weight of glycerol; 13 % by weight of polysorbate 20; 2.0 % by weight of potassium cocoate; 2.0 % by weight of sodium oleate, sorbitol and water, having a pH of about 9-10. The shampoo does not contain preservatives and only contains ingredients approved for use as food additives.

Applicant has now found that polysorbate 20 may be subject to decomposition in the above formulation.

The object of the present invention is to provide a stable baby cleansing formulation which can be formed entirely from constituents approved for use as food additives, *i*.*e*. compounds appearing on the E list. It is a further object to provide a formulation without the need of including a preservative, such as those used in EP-A-0 569 028 and JP 2001 261549A.

The present inventors have now unexpectedly found that by using a combination of polysorbate 20 and sodium stearoyl lactylate at a pH of 4-8, a stable formulation can be obtained, wherein decomposition of the polysorbate 20 is avoided.

Accordingly, for an aqueous formulation of the preamble of claim 1, containing a polyol in an amount of 10-50 % by weight, and polysorbate 20 as main surfactant, this object has been achieved by means of including sodium stearoyl lactylate in an amount of 0.1-2 % by weight of the formulation at a pH of 4-8 of the formulation.

### Summary of invention

The decomposition of polysorbate 20 in the prior art formulation is believed to be due to the high pH value (9-10). The decomposition has been found to produce free fatty acid, which in turn can be deprotonated, leading to a reduced pH of the formulation. At the reduced pH, the fatty acid obtained from decomposition of the polysorbate may precipitate, thus causing a stability problem. The decomposition is also likely to negatively affect the smell of the formulation, especially during long time storage. By using sodium stearoyl lactylate, which is ionized at a lower pH (than the pH of 9-10 of the prior art formulation), and avoiding the potassium cocoate and sodium oleate of the prior art formulation, which require a pH of 9-10, a formulation can be achieved exhibiting a lower pH. The inventors have found polysorbate 20 to be stable at a pH of 4-8, more preferably a pH of 4-7, an optimum pH of the inventive formulation being within the range of pH 5-7.

The present inventors have also found the smell of prior art formulation not to be sufficiently agreeable. Thus, the problem to be solved could in the alternative be regarded as to provide a baby cleansing formulation entirely formed of constituents approved for use as food additives, *i*.*e*. compounds appearing on the E list, having an improved smell. In addition to the decomposition of polysorbate 20 described above, sodium oleate has been identified as a major source of the deficient smell of the formulation. By using sodium stearoyl lactylate as described above, and avoiding the sodium oleate of the prior art formulation, the problem can be solved.

By using the polyol in a concentration as defined in claim 2, a self-preserving formulation can be obtained, not requiring any preservatives such as those used in EP-A-0 569 028 and JP 2001 261549A.

By including sucrose cocoate a better skin feel will be imparted by the formulation. Also, an improved smell will be obtained.

The formulation of the present invention is especially intended for use in products for infants and kids, but may of course also be used by adults.

In one embodiment, the formulation is formulated as a shampoo, preferably containing sorbitol.

In another embodiment, the formulation is formulated as a liquid soap.

Further embodiments and advantages will become apparent from the detailed description and appended claims.

### Detailed description

The use of additives in food items is generally strictly regulated by national and/or international authorities.

In order for an additive to be allowed for use in food, it will generally have to be approved by such authorities. In order for a substance to be allowed for use as a foodstuff additive within e.g. the E.C., it must have been approved for such use by the European Food Safety Authority (EFSA). There is also currently a cooperation on an international level, wherein the Joint FAO/WHO Expert Committee on Food Additives (JECFA) has an important role. If for example the JECFA has found an additive not acceptable, it will be very unlikely for such additive to be accepted within the E.C.

The substances used according to the present invention are substances approved by the EFSA, which substances appear on the so-called E list. The current E list (for the year 2007) is reproduced in Table I, together with four additional substances approved for use as food additives not having an E number. The table is an English translation of the list as published by the Swedish National Food Administration ("Livsmedelsverket") available as "E-nummernyckeln" on the Swedish National Food Administration's web site http://www.slv.se, supplemented with four additional approved substances. The grouping and order of appearance of the additives in the below listing are the same as the list published by Swedish National Food Administration (NFA).

For the purpose of the present invention any reference to the E list shall be taken to refer to the below Table I. More preferably, the E list shall be taken to refer to the substances having an E number, and polyethylene glycol 6000.

**TABLE I (The E list)**

| | |
|---|---|
| **Colours** | E 150a Plain caramel |
| E 100 Curcumin | E 150b Caustic sulphite caramel |
| E 101 (i) Riboflavin (ii) Riboflavin-5'-phosphate | E 150c Ammonia caramel |
| E 102 Tartrazine | E 150d Sulphite ammonia caramel |
| E 104 Quinoline Yellow | E 151 Brilliant Black BN, Black PN |
| | E 153 Vegetable carbon |
| E 110 Sunset Yellow FCF, Orange Yellow S | E 154 Brown FK |
| E 120 Cochineal, Carminic acid, Carmines | E 155 Brown HT |
| E 122 Azorubine, Carmoisine | E 160a Carotenes: (i) Mixed carotenes (ii) Beta-carotene |
| E 123 Amaranth | E 160b Annatto, bixin, norbixin |
| E 124 Ponceau 4R, Cochineal Red A | E 160c Paprika extract, capsanthin, |
| E 127 Erythrosine | capsorubin |
| E 128 Red 2G | E160d Lycopene |
| E 129 Allura Red AC | E 160e Beta-apo-8'-carotenal (C 30) |
| E 131 Patent Blue V | E 160f Ethyl ester of beta-apo-8'-carotenic acid (C 30) |
| E 132 Indigotine, Indigo carmine | E 161b Lutein |
| E 133 Brilliant Blue FCF | E 161g Canthaxanthin |
| E 140 (i) Chlorophylls (ii) Chlorophyllins | E 162 Beetroot Red, betanin |
| E 141 (i) Copper complexes of | E 163 Anthocyanins |
| chlorophylls (ii) Copper complexes of | E 170 Calcium carbonates |
| chlorophyllins | E 171 Titanium dioxide |
| E 142 Greens S | E 172 Iron oxides and hydroxides |
| E 173 Aluminium | E 231 Orthophenyl phenol |
| E 174 Silver | E 232 Sodium orthophenyl phenol |
| E 175 Gold | E 234 Nisin |
| E 180 Litholrubine BK | E 235 Natamycin |
| | E 239 Hexamethylene tetramine |
| **Preservatives** | E 242 Dimethyl dicarbonate |
| E 200 Sorbic acid | E 249 Potassium nitrite |
| E 202 Potassium sorbate | E 250 Sodium nitrite |
| E 203 Calcium sorbate | E 251 Sodium nitrate |
| E 210 Benzoic acid | E 252 Potassium nitrate |
| E 211 Sodium benzoate | E 260 Acetic acid |
| E 212 Potassium benzoate | E 261 Potassium acetate |
| E 213 Calcium benzoate | E 262 Sodium acetates (i) Sodium acetate (ii) Sodium hydrogen acetate (sodium diacetate) |
| E 214 Ethyl p-hydroxybenzoate | E 263 Calcium acetate |
| E 215 Sodium ethyl phydroxybenzoate | E 270 Lactic acid |
| E 216 Propyl p-hydroxybenzoate | E 280 Propionic acid |
| E 217 Sodium propyl phydroxybenzoate | E 281 Sodium propionate |
| E 218 Methyl p-hydroxybenzoate | E 282 Calcium propionate |
| E 219 Sodium methyl phydroxybenzoate | E 283 Potassium propionate |
| E 220 Sulphur dioxide | E 284 Boric acid |
| E 221 Sodium sulphite | E 285 Sodium tetraborate (borax) |
| E 222 Sodium hydrogen sulphite | E 290 Carbon dioxide |
| E 223 Sodium metabisulphite | E 296 Malic acid |
| E 224 Potassium metabisulphite | E 297 Fumaric acid |
| E 226 Calcium sulphite | E 1105 Lysozyme |
| E 227 Calcium hydrogen sulphite | |
| E 228 Potassium hydrogen sulphite | **Antioxidants** |
| E 300 Ascorbic acid | E 334 Tartaric acid (L(+)-) |
| E 301 Sodium ascorbate | E 335 Sodium tartrates (i) Monoso- dium tartrate (ii) Disodium tartrate |
| E 302 Calcium ascorbate | E 336 Potassium tartrates (i) Monopotassium tartrate (ii) Dipotassium tartrate |
| E 304 Fatty acid esters of ascorbic acid (i) Ascorbyl palmitate (ii) Ascor- byl stearate | E 337 Sodium potassium tartrate |
| E 306 Tocopherol-rich extract | E 338 Phosphoric acid |
| E 307 Alpha-tocopherol | E 339 Sodium phosphates (i) Mono- sodium phosphate (ii) Disodium phosphate (iii) Trisodium phosphate |
| E 308 Gamma-tocopherol | E 340 Potassium phosphates (i) Mo- nopotassium phosphate (ii) Dipotas- sium phosphate (iii) Tripotassium phosphate |
| E 309 Delta-tocopherol | E 341 Calcium phosphates (i) Mono- calcium phosphate (ii) Dicalcium phosphate (iii) Tricalcium phosphate |
| E 310 Propyl gallate | E 343 Magnesium phosphates (i) monomagnesium phosphate (ii) Di- magnesium phosphate |
| E 311 Octyl gallate | E 350 Sodium malates (i) Sodium malate (ii) Sodium hydrogen malate |
| E 312 Dodecyl gallate | E 351 Potassium malate |
| E 315 Erythorbic acid | E 352 Calcium malates (i) Calcium malate (ii) Calcium hydrogen malate |
| E 316 Sodium erythorbate | E 353 Metatartaric acid |
| E 320 Butylated hydroxyanisole (BHA) | E 354 Calcium L-tartrate |
| E 321 Butylated hydroxytoluene (BHT) | E 355 Adipic acid |
| E 322 Lecithins | E 356 Sodium adipate |
| E 325 Sodium lactate | E 357 Potassium adipate |
| E 326 Potassium lactate | E 363 Succinic acid |
| E 327 Calcium lactate | E 380 Triammonium citrate |
| E 330 Citric acid | |
| E 331 Sodium citrates (i) Monoso- dium citrate (ii) Disodium citrate (iii) Trisodium citrate | |
| E 332 Potassium citrates (i) Monopo- tassium citrate (ii) Tripotassium citrate | |
| E 333 Calcium citrates (i) Monocal- cium citrate (ii) Dicalcium citrate (iii) Tricalcium citrate | |
| E 385 Calcium disodium ethylene diamine tetra-acetate (Calcium diso- dium EDTA) | E 405 Propan-1,2-diol alginate |
| | E 406 Agar |
| | E 407 Carrageenan |
| **Sweeteners** | E 407a Processed eucheuma seaweed |
| E 420 Sorbitol (i) Sorbitol (ii) Sorbitol syrup | E 410 Locust bean gum |
| E 421 Mannitol | E 412 Guar gum |
| E 950 Acesulfame K | E 413 Tragacanth |
| E 951 Aspartame | E 414 Acacia gum (gum arabic) |
| E 952 Cyclamic acid and its Na and | E 415 Xanthan gum |
| Ca salts | E 416 Karaya gum |
| E 953 Isomalt | E 417 Tara gum |
| E 954 Saccharin and its Na, K and Ca salts | E 418 Gellan gum |
| E 955 Sucralose E | 422 Glycerol |
| E 957 Thaumatin | E 425 Konjac (i) Konjac gum (ii) Konjac glucomannane |
| E 959 NeohesperidineDC | E 431 Polyoxyethylene (40) stearate |
| E 962 Salt of aspartame - acesulfame | E 432 Polyoxyethylene sorbitan monolaurate (polysorbate 20) |
| E 965 Maltitol (i) Maltitol (ii) Maltitol syrup | E 433 Polyoxyethylene sorbitan monooleate (polysorbate 80) |
| E 966 Lactitol | E 434 Polyoxyethylene sorbitan monopalmitate (polysorbate 40) |
| E 967 Xylitol | E 435 Polyoxyethylene sorbitan mo- nostearate (polysorbate 60) |
| **Others** | |
| | E 436 Polyoxyethylene sorbitan tris-tearate (polysorbate 65) |
| E 400 Alginic acid | E 440 Pectins (i) pectin (ii) amidated pectin |
| E 401 Sodium alginate | E 442 Ammonium phosphatides |
| E 402 Potassium alginate | E 444 Sucrose acetate isobutyrate |
| E 403 Ammonium alginate E 403 Ammonium alginate | E 445 Glycerol esters of wood rosins |
| E 404 Calcium alginate | |
| E 450 Diphosphates: (i) Disodium diphosphate (ii) Trisodium diphosphate (iii) Tetrasodium diphosphate (iv) Dipotassium diphosphate (v) Tetrapotassium diphosphate (vi) Di- calcium diphosphate (vii) Calcium dihydrogen diphosphate | E 472b Lactic acid esters of mono and diglycerides of fatty acids |
| | E 472c Citric acid esters of mono and diglycerides of fatty acids |
| | E 472d Tartaric acid esters of mono and diglycerides of fatty acids |
| E 451 Triphosphates: (i) Pentasodium triphosphate (ii) Pentapotassium triphosphate | E 472e Mono- and diacetyl tartaric acid esters of mono- and diglycerides of fatty acids |
| E 452 Polyphosphates: (i) Sodium polyphosphates (ii) Potassium poly-phosphates (iii) Sodium calcium polyphosphate (iv) Calcium polyphophates | E 472f Mixed acetic and tartaric acid esters of mono- and diglycerides of fatty acids |
| | E 473 Sucrose esters of fatty acids |
| E 459 Beta-cyclodextrine | E 474 Sucroglycerides |
| E 460 Cellulose (i) Microcrystalline cellulose (ii) Powdered cellulose | E 475 Polyglycerol esters of fatty acids |
| E 461 Methyl cellulose | E 476 Polyglycerol polyricinoleate |
| E 463 Hydroxypropyl cellulose | E 477 Propane-1,2-diol esters of fatty acids |
| E 464 Hydroxypropyl methyl cellulose | E 479b Thermally oxidized soya bean oil interacted with mono- and diglycerides of fatty acids |
| E 465 Ethyl methyl cellulose | |
| E 466 Carboxy methyl cellulose, Sodium carboxy methyl cellulose | E 481 Sodium stearoyl-2-lactylate |
| E 468 Crosslinked sodium carboxy-methyl cellulose | E 482 Calcium stearoyl-2-lactylate |
| | E 483 Stearyl tartrate |
| E 469 Enzymically hydrolysed car- boxy methyl cellulose | E 491 Sorbitan monostearate |
| | E 492 Sorbitan tristearate |
| E 470a Sodium, potassium and cal- cium salts of fatty acids | E 493 Sorbitan monolaurate |
| E 470b Magnesium salts of fatty acids | E 494 Sorbitan monooleate |
| | E 495 Sorbitan monopalmitate |
| E 471 Mono- and diglycerides of fatty acids | E 500 Sodium carbonates (i) Sodium carbonate (ii) Sodium hydrogen carbonate (iii) Sodium sesquicarbonate |
| E 472a Acetic acid esters of mono- and diglycerides of fatty acids | |

| | |
|---|---|
| E 501 Potassium carbonates (i) Potassium carbonate (ii) Potassium hydrogen carbonate | E 529 Calcium oxide |
| | E 530 Magnesium oxide |
| E 503 Ammonium carbonates (i) Ammonium carbonate (ii) Ammonium hydrogen carbonate | E 535 Sodium ferrocyanide |
| | E 536 Potassium ferrocyanide |
| E 504 Magnesium carbonates (i) Magnesium carbonate (ii) Magnesium hydroxide carbonate | E 538 Calcium ferrocyanide |
| | E 541 Sodium aluminium phosphate, acidic |
| E 507 Hydrochloric acid | E 551 Silicon dioxide |
| E 508 Potassium chloride | E 552 Calcium silicate |
| E 509 Calcium chloride | E 553a (i) Magnesium silicate (ii) Magnesium trisilicate |
| E 511 Magnesium chloride | |
| E 512 Stannous chloride | E 553b Talc |
| E 513 Sulphuric acid | E 554 Sodium aluminium silicate |
| E 514 Sodium sulphates (i) Sodium sulphate (ii) Sodium hydrogen sulphate | E 555 Potassium aluminium silicate |
| | E 556 Calcium aluminium silicate |
| E 515 Potassium sulphates (i) Potassium sulphate (ii) Potassium hydrogen sulphate | E 558 Bentonite |
| | E 559 Aluminium silicate |
| | E 570 Fatty acids |
| E 516 Calcium sulphate | E 574 Gluconic acid |
| E 517 Ammonium sulphate | E 575 Glucono-delta-lactone |
| E 520 Aluminium sulphate | E 576 Sodium gluconate |
| E 521 Aluminium sodium sulphate | E 577 Potassium gluconate |
| E 522 Aluminium potassium sulphate | E 578 Calcium gluconate |
| E 523 Aluminium ammonium sulphate | E 579 Ferrous gluconate |
| | E 585 Ferrous lactate |
| E 524 Sodium hydroxide | E 620 Glutamic acid |
| E 525 Potassium hydroxide | E 621 Monosodium glutamate |
| E 526 Calcium hydroxide | E 622 Monopotassium glutamate |
| E 527 Ammonium hydroxide | E 623 Calcium diglutamate |
| E 528 Magnesium hydroxide | |
| E 624 Monoammonium glutamate | E 943a Butane |
| E 625 Magnesium diglutamate | E 943b Isobutane |
| E 626 Guanylic acid | E 944 Propane |
| E 627 Disodium guanylate | E 948 Oxygen |
| E 628 Dipotassium guanylate | E 949 Hydrogen |
| E 629 Calcium guanylate | E 999 Quilllaia extract |
| E 630 Inosinic acid | E 1103 Invertase |
| E 631 Disodium inosinate | E 1200 Polydextrose |
| E 632 Dipotassium inosinate | E 1201 Polyvinylpyrrolidone |
| E 633 Calcium inosinate | E 1202 Polyvinylpolypyrrolidone |
| E 634 Calcium 5'-ribonucleotides | E 1404 Oxidized starch |
| E 635 Disodium 5'-ribonucleotides | E 1410 Monostarch phosphate |
| E 640 Glycine and its sodium salt | E 1412 Distarch phosphate |
| E 650 Zinc acetate | E 1413 Phosphated distarch phosphate |
| E 900 Dimethyl polysiloxane | |
| E 901 Beeswax, white and yellow | E 1414 Acetylated distarch phosphate |
| E 902 Candelillla wax | E 1420 Acetylated starch |
| E 903 Carnauba wax | E 1422 Acetylated distarch adipate |
| E 904 Shellac | E 1440 Hydroxy propyl starch |
| E 905 Microcrystalline wax | E 1442 Hydroxy propyl distarch phosphate |
| E 907 Hydrogenated poly-1-decene | |
| E 912 Montanic acid esters | E 1450 Starch sodium octenyl succinate |
| E 914 Oxidized polyethylene wax | E 1451 Acetylated oxidised starch |
| E 920 L-Cysteine | E 1505 Triethyl citrate |
| E 927b Carbamide | E 1517 Glyceryl diacetate |
| E 938 Argon | E 1518 Glyceryl triacetate |
| E 939 Helium | E 1519 Benzyl alcohol |
| E 941 Nitrogen | E 1520 Propan-1,2-diol (propylene glycol), |
| E 942 Nitrous oxide | |
| and additionally also the following four substances appearing in the Guidance Document for the NFA's own Code of Statutes, LIVSFS 2004:30, on food additives (19.10.2006). | coagulating enzymes of microbial origin, |
| | - Polyethylene glycol 6000 |
| | - Tertiary-butyl hydroquinone. (TBHQ) |
| - Enzymes and enzyme preparations | |
| - Rennet and rennet pepsin preparations and also milk- | |

The purity of the substances used in the formulation of the inventions is not critical for proper function thereof. Thus, substances of both food grade and cosmetic grade can be used in the inventive formulations. However, while substances of food grade very well can be employed in the formulation of the invention, governmental regulations will typically prescribe that cosmetic grade must be used in cosmetic products, such as the products of the present invention. In some instances, cosmetic grade implies a higher purity than corresponding food grade substance.

### Ingredients

In its most generic form the formulation of the invention comprises 10-50 % by weight of a polyol being glycerol, and optionally a member selected from the group consisting of propylene glycol, sorbitol, mannitol, maltitol, lactitol, and xylitol, or a mixture thereof, polysorbate 20 as main surfactant, and 0.1-2 % by weight of sodium stearoyl lactylate, and water up to 100 %, and exhibits a pH of 4-8.

### Surfactant

The main surfactant is polysorbate 20 (E 432), and is preferably used in an amount of 8-20, more preferably 13-17 % by weight of the formulation. Other polysorbates, such as 80, 40, 60 and 65, should not be present in an amount of more than 2-3 % by weight, if present at all, and are preferably absent, since any presence thereof will have a negative impact on the foam.

### Sodium stearoyl lactylate

Polysorbate 20 does not foam well by itself. The sodium stearoyl lactylate will act as an emulsifier and foam enhancer, and also as a solubilizer of less soluble ingredients, such as the sucrose cocoate used in the present invention. In a preferred embodiment the sodium stearoyl lactylate is present in an amount of 0.1-1 % by weight of the inventive formulation.

### Polyol

When used together with surfactant and foaming agents, the polyol *i*.*a*. improves the foaming properties of the resulting composition.

It has been found that by using high quantities of polyol the product will be self-preserved. In higher quantities it has an antimicrobial activity, since it lowers the water activity in the formulation, so that the there is less water available to micro-organisms to grow in. 30 % by weight of glycerol in the formulation is presently considered to be a suitable amount for achieving the desired antimicrobial effect. By using other polyols selected from the group consisting of propylene glycol, sorbitol, mannitol, maltitol, lactitol, and xylitol, or a mixture thereof, in addition to glycerol, it has been found that the amount of polyol requried for self-preservation can be reduced to 20 % by weight. An especially preferred polyol in this regard is propylene glycol.

Accordingly, in one embodiment the inventive formulation is self-preserved by means of presence of a sufficient amount of polyol.

### Sucrose cocoate

Addition of sodium cocoate to the formulation will impart a better skin feel after use of the formulation. The sodium cocoate will act as emollient. Also, sodium cocoate will improve the smell of the formulation. Sodium cocoate is preferably included in an amount of 0.5-2.5 %, more preferably 0.5-1.5 %, and especially 0.7-1.4 %, by weight of the formulation.

### Polyethylene glycol 6000

In order to further improve the foam stability polyethylene glycol 6000 can be included in the formulation. The polyethylene glycol 6000 is preferably included in an amount of 0.5-8, preferably 2-6, more preferably 2-4 % by weight of the formulation.

### Sorbitol

When the formulation is formulated as a shampoo it is preferred that sorbitol is included for imparting foaming, and hair improving properties, such as better combing properties. Sorbitol is preferably included in an amount of 0.25-2 % by weight.

### Viscosity

The formulation of the present invention is entirely liquid in itself, *i*.*e*. of a low viscosity. Therefore, in order to be of practical use, the formulation should be brought into a more convenient viscosity. Also, the formulation in itself may be difficult to foam properly. Especially the presence of glycerol, particularly when used in higher amounts, such as in the high content required for self-preservation, has been found to tend to make it more difficult to increase the viscosity of the formulation. Presently known thickening agents appearing on the E-list have been found to be inefficient for the purpose of increasing the viscosity of the formulation. These drawbacks can be readily overcome by means of using a foaming dispenser, which brings the formulation into a foam when dispensed. Such dispensers are commercially available. Accordingly, the soap or shampoo formulation can be contained in a suitable container, which container is provided with a foaming dispenser, such as foaming pump. Suitable foaming pumps, also referred to as pump foamers or foam pumps, are e.g. commercially available from Airspray N.V. (Alkmaar, The Netherlands).

### pH of the formulations

The inventive formulation should exhibit a pH within the interval of 4-8 in order for the formulation to exhibit desired stability. Accordingly, in a preferred embodiment of the invention a buffer system is included in the formulation. When used, the buffer system will be included in the inventive formulation in an amount effective for buffering the formulation at the desired pH value. Suitable buffers are citric acid, lactic acid, phosphoric acid, malic acid, tartaric acid and sodium carbonate.

### Other ingredients appearing on the E list

Any soap appearing on the E list which can be stably dissolved at the pH of the present formulation could be present in the formulation. Thus, sodium oleate and potassium cocoate should be avoided. For the purpose of the E list, a soap is defined as being a salt of a free fatty acid (e.g. E 470a).

Other ingredients appearing on the E list could also be included, such as gelling agents, consistency agents, and modified starch, and gums, such as for example gellan (E 418), guar (E 412), karaya (E 416), xanthan (E 415) and arabicum (E 414), especially xanthan gum and gellan gum.

### Other edible substances

According to the present invention it is also conceivable to include other edible substances in the formulation. As used in the present disclosure the term "other edible substances" is intended to refer to substances not appearing on the E list, which substances are used in food stuffs, and which could be added to the inventive formulation for added comfort to the person using the formulation, or for making the formulation more attractive to the consumer, or for increasing the viscosity thereof, without jeopardizing the edibility and intended function of the formulation. Such substances are however entirely optional and are not critical as long as the edibility and/or the intended function of the formulation is not jeopardized. Such substances can for example be vegetable oils mainly consisting of mixtures of triglycerides, starch and gums and/or aromas and fragrances.

Examples of gums which could optionally be included according to the invention are acacia catechu, acacia farnesina, acacia senegal, astragalus gummifer, boswella serrata, caesalpina spinosa, callitris quadrivalvis, cellulose, natto, rhizobian, sclerotium, and welan.

Examples of starches which could optionally be included according to the invention are arrowroot, tapioca, potato, sweet potato, bean (fava, lentile, pea), arracacha, buckwheat, banana, barley, cassava, corn, kudzu, oca, rice, sago, sorghum, taro, wheat, and yams.

Specific examples of vegetable oils which could optionally be included according to the invention are almond oil, apricotkernel oil, bilberry seed oil, black cummin oil, black currant seed oil, cacao butter, canola oil, cloudberry seed oil, coconut oil, corn oil, cottonseed oil, cranberry seed oil, elaeis guineensis oil, garlic oil, glycine soya oil, grapeseed oil, groundnut oil, hazelnut oil, hemp oil, lingonberry seed oil, linseed oil, macadamianut oil, mango seed oil, marine oil, olive oil, palm oil, palm kernel oil, peachkernel oil, peanut oil, pistachionut oil, poppyseed oil, pumpkinseed oil, rapeseed oil, raspberry seed oil, rice germ oil, safflower oil, sea bucktorn berry oil, sea bucktron seed oil, sesame oil, shea oil, shea butter, shorea stenoptera butter, soybean oil, strawberry seed oil, sunflower oil, walnut oil, and wheatgerm oil.

The group of aromas/fragrances embraces all substances of natural, natural identical (synthetically derived) and/or synthetic origin, added with the intention of influencing the smell or making the product more attractive. Examples of aromas/fragrances are both complex blends simulating the taste of fruits, such as strawberry, citrus and/or melon or single substances such as vanillin.

Specific examples of fragrances and aromas, typically in the form of oils or extracts, which could optionally be included according to the invention are those derived from barley, oat, caraway seed, birch bud, juniper sprout, capers, garlic oil, glutamat, mango-chutney, black pepper oil, sambal, soy, paprika, capsicum, ginger oil, nutmeg, mace, turmeric, coriander oil, menthol, eukalypthus, ajowan oil, betel leaf oil, cardamom oil, caraway oil, celery seed oil, cinnamon oil, cinnamon bark oil, cinnamon leaf oil, clove bud oil, cumin seed oil, cubeb oil, dill seed oil, fennel seed oil, hing oil, mace oil, nut meg oil, onion oil, star anise oil, turmeric oil, ambrette seed oil, angelica root oil, aromise oil, amyris oil, bay oil, bergamot oil, birch tar oil, basil oil, basil oil, basil oil, bois - de - rose oil, bursera oil, cade oil, cajiput oil, camphor oil, cananga oil, cassia oil, clary sage oil, curry leaf oil, calamus oil, costus root oil, carrot seed oil, cedar wood oil, citronella java oil, cypress oil, cyperiol oil, chamomile blue oil, chamomile roman oil, combava oil, davana oil, elemi oil, eucalyptus citrodora oil, eucalyptus globules oil, frank incense oil, geranium oil, ginger grass oil, ginger lily oil, galangal oil, gurjam oil, grape fruit oil, hope oil, hyssop oil, immortelle oil, juniper berry oil, juniper leaf oil, jasmine, kapoor katcheri oil, lavender oil, lemon oil, lemon grass oil, lemon melissa oil, lime oil, laural berry oil, lemon balm oil, litsea cubeba oil, lovage oil, mentha citrata oil, mentha piperata oil, mentha shivalik oil, mandarin oil, marjoram oil, mimosa absolute, myrtile oil, nar kachur oil, neroli oil, naiouli oil, orange oil, patchouli oil, petit green oil, pine oil, palma rosa oil, pimento berry oil, rose wood oil, rose oil indian, rose marry oil, sage oil, sugandh mantri oil, sugandh kokila oil, spike nard oil, tarragon oil, tangerine oil, tea tree oil, thuja wood oil, tomar seed oil, tagettues oil, tuberose absolute, vetiver oil, valerian root oil, verbina oil, vervain oil, winter green oil, worm wood oil, ylang ylang oil, yarrow oil, and zadoeria oil.

Some of the above-listed fragrances and aromas could also be included as skin-conditioning agents.

### Preservatives

In an alternative embodiment the formulation of the invention could also contain a preservative. Thereby, the high polyol content used for self-preservation will not be necessary, and the content of polyol can consequently be reduced. Suitable preservatives are benzoic acid, sorbic acid, and derivatives thereof, such as the sodium and calcium salts. However, from an allergenic view point the use of preservatives is not preferred, since preservatives are believed to be allergenic. Especially for the purpose of products intended for infants and kids, preservatives should be avoided.

### Preparation

The formulations can be prepared by merely mixing the ingredients together in any order of addition, especially since all the ingredients are water soluble. Heating may be of advantage but is not necessary. Powdery components may be heated in (a sufficient fraction of) the polyol in order to enhance the solution process thereof. Especially water-swellable ingredients, such as starch and gum, if present, are conveniently first dispersed in the polyol, or a sufficient fraction thereof, and thereafter mixed with the water. Thereby, wetting and swelling by the water-swellable ingredients will only take place after dispersion thereof. By doing so, any homogenization of the mixture may be omitted, which otherwise is likely to be required due to possible agglomeration of the water-swellable ingredients upon contact with water.

The below examples are provided for illustrative purposes only, without limiting the invention thereto.

### Examples

### Example 1 - Baby Wash - a preservative free liquid soap formulation

| | |
|---|---|
| Aqua | 51,3 |
| Glycerine | 15,0 |
| Propylene Glycol | 15,0 |
| Polysorbate 20 | 15,0 |
| PEG-150* | 2,0 |
| Sucrose Cocoate | 0,7 |
| Sodium Stearolyl Lactylate | 0,5 |
| Sodium Citrate | 0,5 |

| | |
|---|---|
| *PEG 150 is the corresponding INCI name of polyethylene glycol 6000. | |

The above components were mixed together, thereby obtaining the inventive formulation.

The soap formulation exhibited long-term stability, and foamed well when dispensed from a foaming pump. The smell was found to be most agreeable.

### Example 2 - Preservative free Baby Shampoo

| | |
|---|---|
| Aqua | 50,6 |
| Glycerine | 15,0 |
| Propylene Glycol | 15,0 |
| Polysorbate 20 | 15,0 |
| PEG-150* | 2,0 |
| Sucrose Cocoate | 0,7 |
| Sorbitol | 0,7 |
| Sodium Stearolyl Lactylate | 0,5 |
| Sodium Citrate | 0,5 |

| | |
|---|---|
| *PEG 150 is the corresponding INCI name of polyethylene glycol 6000. | |

The above components were mixed together, thereby obtaining the inventive formulation.

The shampoo formulation exhibited long-term stability, and foamed well when dispensed from a foaming pump. The smell of the formulation was found to be most agreeable.

## Claims

1. A cleansing formulation for personal use which can be formed entirely from substances appearing in Table I and water, comprising a polyol in an amount of 10-50 % by weight and polysorbate 20 as main surfactant, and optionally, other edible substances used in food, **characterised in that** the polyol is glycerol, and optionally a member selected from the group consisting of propylene glycol, sorbitol, mannitol, maltitol, lactitol, and xylitol, or a mixture thereof, and **in that** the formulation additionally contains 0.1-2 % by weight of sodium stearoyl lactylate, and **in that** the formulation exhibits a pH of 4-8.

2. The cleansing formulation of claim 1, **characterised in that** the polyol is contained in a total amount of 20-50 % by weight of the formulation, with the proviso that when only glycerol is used as the polyol then glycerol is present in an amount of 30-50 % by weight of the formulation.

3. The cleansing formulation of claim 1 or 2, containing sucrose cocoate in an amount of 0.5-2.5, preferably 0.5-1.5, and more preferably 0.7-1.4 % by weight.

4. The cleansing formulation of any of the previous claims, containing polyethylene glycol 6000 in an amount of 0.5-8, preferably 2-6, more preferably 2-4 % by weight.

5. The cleansing formulation of any of the previous claims, containing polysorbate 20 in an amount of 8-20, more preferably 13-17 % by weight.

6. The cleansing formulation of any of the previous claims, containing propylene glycol and glycerol, preferably in a ratio of about 1:1, preferably in a total amount of 20-50 % by weight of the formulation.

7. The cleansing formulation of any of the previous claims formulated as a shampoo product, preferably additionally containing sorbitol.

8. The cleansing formulation of any of the claims 1-6, formulated as a liquid soap product.

9. The cleansing formulation of any of the previous claims, additionally containing a buffering agent.

10. The cleansing formulation of any of the previous claims containing only substances appearing in Table I and water.

11. Method of preparing the formulation of any of the previous claims, wherein any water-swellable ingredients are first dispersed in the polyol, or a sufficient fraction thereof, and thereafter mixed with the water.

## Patentansprüche

1. Eine Reinigungsformulierung zum persönlichen Gebrauch, die vollständig aus Substanzen, die in Tabelle 1 vorkommen, und Wasser gebildet werden kann, umfassend ein Polyol in einer Menge von 10 bis 50 Gewichts-% und Polysorbat 20 als hauptsächliches grenzflächenaktives Mittel und optional andere essbare, in Lebensmitteln verwendete Substanzen, **dadurch gekennzeichnet, dass** das Polyol Glycerin und optional ein Mitglied, ausgewählt aus der Gruppe bestehend aus Propylenglykol, Sorbit, Mannit, Maltit, Lactit und Xylit oder einem Gemisch davon, ist und dass die Formulierung zusätzlich 0,1 bis 2 Gewichts-% Natriumstearoyllactylat enthält und dass die Formulierung einen pH-Wert von 4 bis 8 aufweist.

2. Die Reinigungsformulierung gemäß Anspruch 1, **dadurch gekennzeichnet, dass** das Polyol in einer Gesamtmenge von 20 bis 50 Gewichts-% der Formulierung enthalten ist, mit der Maßgabe, dass, wenn nur Glycerin als Polyol verwendet wird, dann Glycerin in einer Menge von 30 bis 50 Gewichts-% der Formulierung vorhanden ist.

3. Die Reinigungsformulierung gemäß Anspruch 1 oder 2, die Sucrosecocoate in einer Menge von 0,5 bis 2,5, bevorzugt 0,5 bis 1,5 und stärker bevorzugt 0,7 bis 1,4 Gewichts-%, enthält.

4. Die Reinigungsformulierung gemäß einem der vorherigen Ansprüche, die Polyethylenglykol 6000 in einer Menge von 0,5 bis 8, bevorzugt 2 bis 6, stärker bevorzugt 2 bis 4 Gewichts-%, enthält.

5. Die Reinigungsformulierung gemäß einem der vorherigen Ansprüche, die Polysorbat 20 in einer Menge von 8 bis 20, stärker bevorzugt 13 bis 17 Gewichts-%, enthält.

6. Die Reinigungsformulierung gemäß einem der vorherigen Ansprüche, die Propylenglykol und Glycerin, bevorzugt in einem Verhältnis von etwa 1:1, bevorzugt in einer Gesamtmenge von 20 bis 50 Gewichts-% von der Formulierung, enthält.

7. Die Reinigungsformulierung gemäß einem der vorherigen Ansprüche, formuliert als ein Haarwaschmittelprodukt, das bevorzugt zusätzlich Sorbit enthält.

8. Die Reinigungsformulierung gemäß einem der Ansprüche 1 bis 6, formuliert als ein Flüssigseifenprodukt.

9. Die Reinigungsformulierung gemäß einem der vorherigen Ansprüche, die zusätzlich ein Pufferungsmittel enthält.

10. Die Reinigungsformulierung gemäß einem der vorherigen Ansprüche, die nur Substanzen, die in Tabelle 1 vorkommen, und Wasser enthält.

11. Verfahren zur Herstellung der Formulierung gemäß einem der vorherigen Ansprüche, wobei jegliche wasserquellbaren Bestandteile oder ein ausreichender Teil davon zuerst im Polyol dispergiert und anschließend mit dem Wasser gemischt werden.

## Revendications

1. Formulation de nettoyage pour une utilisation personnelle qui peut être entièrement formée à partir de substances présentées dans le tableau I et de l'eau, comprenant un polyol en une quantité de 10 à 50 % en poids et du polysorbate 20 en tant que tensioactif principal et, éventuellement, d'autres substances comestibles utilisées dans des aliments, **caractérisée en ce que** le polyol est le glycérol et, éventuellement, un membre choisi dans le groupe constitué du propylène glycol, du sorbitol, du mannitol, du maltitol, du lactitol et du xylitol, ou un mélange de ceux-ci, et **en ce que** la formulation comprend en plus 0,1 à 2 % en poids de stéaroyl-lactylate de sodium et **en ce que** la formulation présente un pH de 4 à 8.

2. Formulation de nettoyage selon la revendication 1, **caractérisée en ce que** le polyol est contenu en une quantité totale de 20 à 50 % en poids de la formulation, à condition que lorsqu'uniquement du glycérol est utilisé comme polyol, alors le glycérol est présent en une quantité de 30 à 50 % en poids de la formulation.

3. Formulation de nettoyage selon la revendication 1 ou 2, contenant du cocoate de saccharose en une quantité de 0,5 à 2,5, de préférence de 0,5 à 1,5, et mieux encore de 0,7 à 1,4 % en poids.

4. Formulation de nettoyage selon l'une quelconque des revendications précédentes, contenant du polyéthylène glycol 6000 en une quantité de 0,5 à 8, de préférence de 2 à 6, mieux encore de 2 à 4 % en poids.

5. Formulation de nettoyage selon l'une quelconque des revendications précédentes, contenant du polysorbate 20 en une quantité de 8 à 20, mieux encore de 13 à 17 % en poids.

6. Formulation de nettoyage selon l'une quelconque des revendications précédentes, contenant du propylène glycol et du glycérol, de préférence dans un rapport d'environ 1:1, de préférence en une quantité totale de 20 à 50 % en poids de la formulation.

7. Formulation de nettoyage selon l'une quelconque des revendications précédentes, formulée sous forme d'un shampoing, de préférence contenant en plus du sorbitol.

8. Formulation de nettoyage selon l'une quelconque des revendications 1 à 6, formulée sous forme d'un savon liquide.

9. Formulation de nettoyage selon l'une quelconque des revendications précédentes, contenant en plus un agent tampon.

10. Formulation de nettoyage selon l'une quelconque des revendications précédentes, contenant uniquement les substances présentées dans le tableau I et de l'eau.

11. Procédé de préparation de la formulation selon l'une quelconque des revendications précédentes, dans lequel tout ingrédient susceptible de se dilater dans l'eau est d'abord dispersé dans le polyol, ou une fraction suffisante de celui-ci, et par la suite mélangé avec l'eau.
